# EUROPEAN PATENT SPECIFICATION

(11) **EP 4 177 257 B1**
(45) Date of publication and mention of the grant of the patent: **10.12.2025**
(21) Application number: 21837478.3
(22) Date of filing: 05.07.2021
(51) Int. Cl.: C07D 495/04, A61K 31/4365, A61P 5/10, A61P 25/00, A61P 25/16

(54) **SUCCINATE OF OCTAHYDROTHIENOQUINOLINE COMPOUND, AND CRYSTALS THEREOF**
SUCCINAT EINER OCTAHYDROTHIENOCHINOLINVERBINDUNG UND KRISTALLE DAVON
SUCCINATE DE COMPOSÉ OCTAHYDROTHIÉNOQUINOLINE ET CRISTAUX DE CELUI-CI

(30) Priority: 06.07.2020 JP 2020116507
(43) Date of publication of application: 10.05.2023
(73) Proprietor: Kissei Pharmaceutical Co., Ltd., Matsumoto-Shi, Nagano 399-8710 (JP)
(72) Inventor: TAKEUCHI, Hideki, Azumino-shi, Nagano 399-8304 (JP); JO, Kazumichi, Joetsu-shi, Niigata 942-0145 (JP)
(74) Representative: Boult Wade Tennant LLP
(86) International application number: PCT/JP2021/025233
(87) International publication number: WO 2022/009815

(56) References cited:
- WO-A1-2012/124649
- JP-A- 2014 074 013
- JP-A- 2014 088 362
- "Polymorphism and crystallization of the pharmaceutical drugs", 20 September 2002, ISBN: 4-901689-06-1, article ASHIZAWA, KAZUHIDE: "Table of content", pages: 1 - 13, XP009541519
- "Saishin Soyaku Kagaku last volume TECHNOMICS [THE PRACTICE OF MEDICINAL CHEMISTRY], C.G. Wermuth (ed.)", vol. 2, 25 September 1999, TEKUNOMIKKU , JP , ISBN: 4-924746-80-0, article BRADLEY D. ANDERSON; KARL P. FLORA: "Passage; Chapter 34: Preparation of water-soluble organic compounds by salt formation", pages: 347 - 354, XP009541633

## Description

### Technical Field

The present invention relates to a succinate salt of 1-{[(4aR,6R,8aR)-2-amino-3-cyano-8-methyl-4,4a,5,6,7,8,8a,9-octahydrothieno[3,2-g]quinolin-6-yl]carbonyl}-3-[2-(dimethylamino)ethyl]-1-propylurea (hereinafter, sometimes referred to as "succinate salt of the present invention") which has dopamine D2 receptor agonist action and which is useful as an agent for the prevention or treatment of Parkinson's disease, restless legs syndrome, hyperprolactinemia or the like.

### Background Art

A compound represented by the formula: 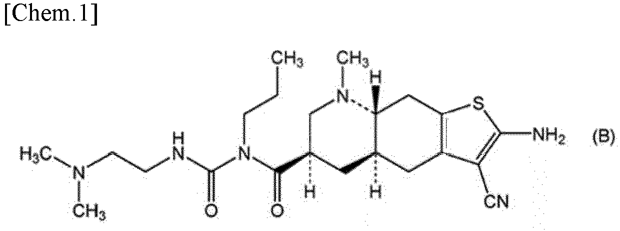 (chemical name: 1-{[(4aR,6R,8aR)-2-amino-3-cyano-8-methyl-4,4a,5,6,7,8,8a,9-octahydrothieno[3,2-g]quinolin-6-yl]carbonyl}-3-[2-(dimethylamino)ethyl]-1-propylurea; hereinafter, sometimes referred to as a "compound (B)") or a hydrochloride salt thereof that has dopamine D2 receptor agonist action and is useful as an agent for the prevention or treatment of Parkinson's disease, restless legs syndrome, hyperprolactinemia or the like is disclosed in Patent literatures 1 to 3. However, the succinate salt of the compound (B) is described only as a general salt, and the characteristics of the succinate salt of the compound (B) are not reported at all.

### Citation List

### Patent Literature

Patent literature 1: International publication No.WO2012/124649 pamphlet.
Patent literature 2: Japanese patent publication No.2014-088362 gazette.
Patent literature 3: Japanese patent publication No.2014-073013 gazette.

### Summary of the Invention

### Problems to be Solved by the Invention

As a result of intensive studies by the present inventors, the hydrochloride salt of the compound (B) described in Patent literatures 1 to 3 is unstable to heat and has poor storage stability due to a crystal form transition caused by high hygroscopicity as described in the stability tests of Test Examples 4 and 5 below, and it is necessary to improve physical properties for use as a drug substance.

An object of the present invention is to provide a different form of the compound (B) which has high storage stability and is suitable for use as a drug substance.

### Means for Solving the Problems

As a result of intensive studies over the foregoing problems, the present inventors found that a succinate salt of 1-{[(4aR,6R,8aR)-2-amino-3-cyano-8-methyl-4,4a,5,6,7,8,8a,9-octahydrothieno[3,2-g]quinolin-6-yl]carbonyl}-3-[2-(dimethylamino)ethyl]-1-propylurea has excellent storage stability and excellent crystallinity, is suitable for industrial production and thus is a suitable compound as a drug substance, and the inventors completed the present invention.

That is, the present invention relates to the following [1] to [17] and the like.
[1] A succinate salt of 1-{[(4aR,6R,8aR)-2-amino-3-cyano-8-methyl-4,4a,5,6,7,8,8a,9-octahydrothieno[3,2-g]quinolin-6-yl]carbonyl}-3-[2-(dimethylamino)ethyl]-1-propylurea.
[2] The succinate salt according to the above [1] represented by the following formula (A-1) or formula (A-2). 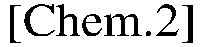 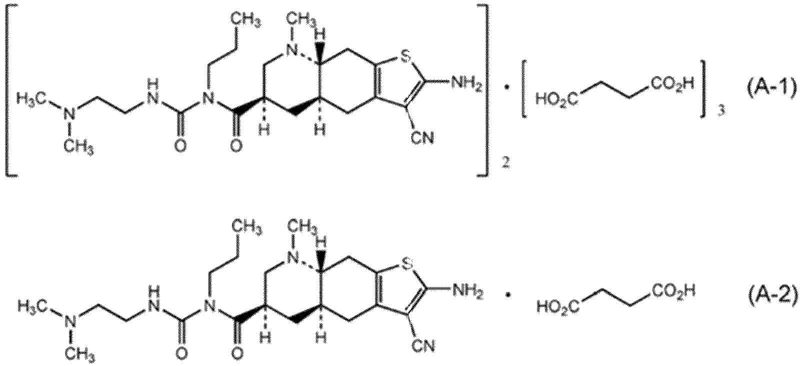
[3] The succinate salt according to the above [1] represented by the following formula (A-1). 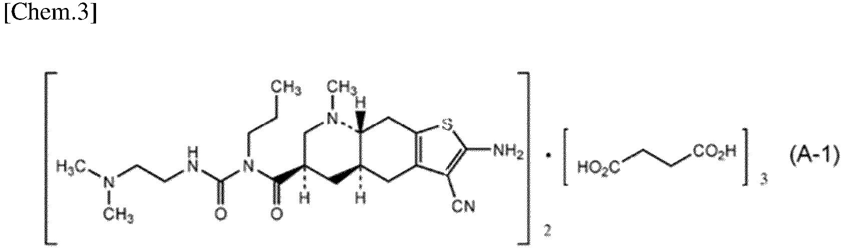
[4] The succinate salt according to the above [1] represented by the following formula (A-2). 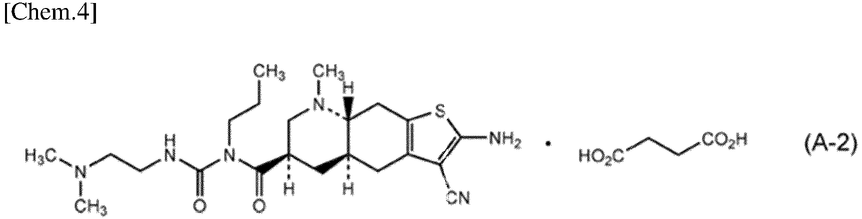
[5] The succinate salt according to the above [3], which is crystalline.
[6] The succinate salt according to the above [4], which is crystalline.
[7] The succinate salt according to the above [5], which has peaks at diffraction angles (2*θ* (°)) of 11.2±0.3 and 11.8±0.3 in a powder X-ray diffraction diagram.
[8] The succinate salt according to the above [5], which is characterized by having an endothermic peak at around 150°C in a thermogravimetric-differential thermal analysis chart.
[9] The succinate salt according to the above [5], which has peaks at chemical shift values (δ (ppm)) of 183.6±0.5, 180.5±0.5, 174.1±0.5, 170.0±0.5, 165.1±0.5 and 157.3±0.5 in a ¹³C solid-state NMR spectrum chart.
[10] The succinate salt according to the above [5], which is characterized by 2 or 3 physical features selected from the group consisting of the following (a1) to (a3):
   (a1) a powder X-ray diffraction diagram having peaks at diffraction angles (2*θ* (°)) of 11.2±0.3 and 11.8±0.3;
   (a2) a ¹³C solid-state NMR spectrum chart having peaks at chemical shift values (δ (ppm)) of 183.6±0.5, 180.5±0.5, 174.1±0.5, 170.0±0.5, 165.1±0.5 and 157.3±0.5; and
   (a3) a thermogravimetric-differential thermal analysis chart having an onset temperature of an endothermic peak at around 150°C.
[11] The succinate salt according to the above [6], which has peaks at diffraction angles (2*θ* (°)) of 8.3±0.3, 12.4±0.3, 15.6±0.3 and 23.2±0.3 in a powder X-ray diffraction diagram.
[12] The succinate salt according to the above [6], which has peaks at chemical shift values (δ (ppm)) of 177.7, 176.4, 166.2, 160.4, 154.0 and 152.4 in a ¹³C solid-state NMR spectrum chart.
[13] A pharmaceutical composition comprising the succinate salt according to any of the above [1] to [12].
[14] The pharmaceutical composition according to the above [13], for use in a method for the treatment or prevention of Parkinson's disease, restless legs syndrome or hyperprolactinemia.
[15] A use of the succinate salt according to any of the above [1] to [12], which is for the manufacture of an agent for the treatment or prevention of Parkinson's disease, restless legs syndrome or hyperprolactinemia.
[16] A succinate salt for use in a method for the treatment or prevention of Parkinson's disease, restless legs syndrome or hyperprolactinemia, which is characterized by administering an effective amount of the succinate salt according to any of the above [1] to [12].
[17] A pharmaceutical composition comprising the succinate salt according to the above [1] having peaks at diffraction angles (2*θ* (°)) of 11.2±0.3 and 11.9±0.3 in a powder X-ray diffraction diagram of the pharmaceutical composition and at least one additional excipient.
[18] A pharmaceutical composition comprising the succinate salt according to the above [1] having peaks at chemical shift values (δ (ppm)) of 183.5±0.5 and 180.4±0.5 in a ¹³C solid-state NMR spectrum chart of the pharmaceutical composition and at least one additional excipient.

### Effect of the Invention

The succinate salt of the present invention is excellent in storage stability because it does not absorb moisture during long-term storage and hardly exhibits a decrease in purity. Further, the succinate salt has excellent solubility, crystallinity and excellent fluidity and thus is a compound which is easy to handle in formulation, for example.

### Brief Description of Drawings

[Figure 1] Figure 1 is a powder X-ray diffraction diagram of the Form I crystal of the salt (A-1). The vertical axis shows X-ray diffraction intensity (Counts), and the horizontal axis shows diffraction angle (2*θ* (°)).
[Figure 2] Figure 2 is a powder X-ray diffraction diagram of the Form II crystal of the salt (A-1). The vertical axis shows X-ray diffraction intensity (Counts), and the horizontal axis shows diffraction angle (2*θ* (°)).
[Figure 3] Figure 3 is a powder X-ray diffraction diagram of the Form I crystal of the salt (A-2). The vertical axis shows X-ray diffraction intensity (Counts), and the horizontal axis shows diffraction angle (2*θ* (°)).
[Figure 4] Figure 4 is a thermogravimetric-differential thermal analysis chart (TG-DTA measurement diagram) of the Form I crystal of the salt (A-1). The vertical axis (left) shows weight (%) in a thermogravimetric (TG) curve, the vertical axis (right) shows heat flux (µv) in a differential thermal analysis (DTA) curve, and the horizontal axis shows temperature (°C).
[Figure 5] Figure 5 is a DSC measurement diagram of the Form I crystal of the salt (A-1). The vertical axis (left) shows weight (%) in a thermogravimetric (TG) curve, the vertical axis (right) shows heat flux (µV) in a differential thermal analysis (DTA) curve, and the horizontal axis shows temperature (°C).
[Figure 6] Figure 6 is a ¹³C solid-state NMR spectrum chart of the Form I crystal of the salt (A-1). The vertical axis shows intensity, and the horizontal axis shows chemical shift value (δ (ppm)).
[Figure 7] Figure 7 is a ¹³C solid-state NMR spectrum chart of the Form I crystal of the salt (A-2). The vertical axis shows intensity, and the horizontal axis shows chemical shift value (δ (ppm)).
[Figure 8] Figure 8 is a powder X-ray diffraction diagram of the hydrochloride salt obtained in Comparative Example 1. The vertical axis shows X-ray diffraction intensity (Counts), and the horizontal axis shows diffraction angle (2*θ* (°)).
[Figure 9] Figure 9 is a powder X-ray diffraction diagram of the crystal of a sebacate salt obtained in Comparative Example 2. The vertical axis shows X-ray diffraction intensity (Counts), and the horizontal axis shows diffraction angle (2*θ* (°)).
[Figure 10] Figure 10 is a powder X-ray diffraction diagram of the crystal of an adipate salt obtained in Comparative Example 3. The vertical axis shows X-ray diffraction intensity (Counts), and the horizontal axis shows diffraction angle (2*θ* (°)).
[Figure 11] Figure 11 is a water adsorption and desorption isotherm of the Form I crystal of the salt (A-1). The solid line indicates the adsorption isotherm, and the broken line indicates the desorption isotherm. The vertical axis shows mass change (%), and the horizontal axis shows relative humidity (% RH).
[Figure 12] Figure 12 is a water adsorption and desorption isotherm of the hydrochloride salt obtained in Comparative Example 1. The solid line indicates the adsorption isotherm, and the broken line indicates the desorption isotherm. The vertical axis shows mass change (%), and the horizontal axis shows relative humidity (% RH).
[Figure 13] Figure 13 is a water adsorption and desorption isotherm of the crystal of a sebacate salt obtained in Comparative Example 2. The solid line indicates the adsorption isotherm, and the broken line indicates the desorption isotherm. The vertical axis shows mass change (%), and the horizontal axis shows relative humidity (% RH).
[Figure 14] Figure 14 is a powder X-ray diffraction diagram of the pharmaceutical composition obtained in Example 4. The vertical axis shows X-ray diffraction intensity (Counts), and the horizontal axis shows diffraction angle (2*θ* (°)).
[Figure 15] Figure 15 is a ¹³C solid-state NMR spectrum chart of the pharmaceutical composition obtained in Example 4. The vertical axis shows intensity, and the horizontal axis shows chemical shift value (δ (ppm)).

### Mode for Carrying out the Invention

Hereinafter, embodiments of the present invention are described in more detail.

The succinate salt of the present invention can be produced, for example, using the following method. Specifically, for example, it can be produced by mixing the compound (B) which can be produced using the method described in Patent literature 1 or using methods according to this method and 0.5 to 2 equivalents of succinic acid in a suitable solvent, dissolving under heating, then concentrating or adding a solvent as appropriate as required and isolating the succinate salt precipitated by cooling. Furthermore, the succinate salt may be purified by recrystallization using the same or similar solvent.

The good solvent may be any solvent, provided that it does not interfere with salt formation, and for example, alcohols such as methanol, ethanol, 1-propanol, 2-propanol, 1-butanol, 2-butanol and the like, tetrahydrofuran, dimethylsulfoxide, N,N-dimethylformamide, N,N-dimethylacetamide and the like can be used. Also, two or more kinds of good solvents of alcohols such as methanol, ethanol, 1-propanol, 2-propanol, 1-butanol, 2-butanol and the like, ethers such as tetrahydrofuran, 1,4-dioxane and the like, solvents such as acetone, acetonitrile, dimethylsulfoxide, N,N-dimethylformamide, N,N-dimethylacetamide and the like and water may be used in combination.

As a poor solvent that can be appropriately added to the good solvent after salt formation, carboxylic acid esters such as methyl acetate, ethyl acetate, isopropyl acetate and the like, ketones such as acetone, methyl ethyl ketone, methyl isobutyl ketone and the like, ethers such as tetrahydrofuran, 1,4-dioxane and the like, acetonitrile, toluene or the like can be used. In addition, two or more kinds of poor solvents may be used in combination.

The succinate salt of the present invention can be purified by recrystallization of the succinate salt produced according to the above-described method or the like using an appropriate recrystallization solvent such as an acetone-water mixed solvent, a methanol-water mixed solvent, an ethanol-water mixed solvent, dimethyl sulfoxide and the like, as necessary.

The succinate salt of the present invention also includes a salt co-crystal with succinic acid, a co-crystal with succinic acid, a hydrate or a solvate with a pharmaceutically acceptable solvent such as ethanol and the like.

The succinate salt of the present invention has dopamine D2 receptor agonist action and is useful as an agent for the prevention or treatment of Parkinson's disease, restless legs syndrome, hyperprolactinemia or the like.

The pharmaceutical composition of the present invention comprises the succinate salt of the present invention as an active ingredient.

These pharmaceutical compositions can be produced depending on their formulations optionally by admixing an appropriate pharmaceutical additive such as excipients, disintegrators, binders, lubricants and the like in accordance with conventional pharmaceutical methods and formulating the mixture in accordance with conventional methods.

For example, powders can be formulated by, if desired, admixing well an active ingredient with appropriate excipients, lubricants and the like. For example, tablets can be formulated by tableting an active ingredient with appropriate excipients, disintegrators, binders, lubricants and the like in accordance with conventional methods. Furthermore, if desired, tablets can be suitably coated to provide film-coated tablets, sugar-coated tablets, enteric-coated tablets and the like. For example, capsules can be formulated by admixing well an active ingredient with appropriate excipients, lubricants and the like or formulating granules or fine granules in accordance with conventional methods and then filling it in appropriate capsules. Furthermore, in the case of such an oral administration preparation, the preparation can also be quick-release or sustained-release formulation depending on the prevention or treatment methods.

When the pharmaceutical composition of the present invention is employed in the practical prevention or treatment, the dosage of the succinate salt of the present invention as the active ingredient is appropriately decided depending on the age, sex, body weight, degree of disorders and treatment of each patient and the like. However, for example, the dosage is approximately within the range of from 0.1 to 300 mg per day per adult human in the case of oral administration, and the dose or several divided doses thereof can be administered. Preferably, the above pharmaceutical composition is produced in such a way that the succinate salt of the present invention is administered in the range of from 0.1 to 300 mg per day per adult human in the case of orally-administered agents.

It is common knowledge that the relative intensity of each peak (relative peak height) in powder X-ray diffraction patterns may fluctuate depending on the sample conditions, the measurement conditions or the measurement apparatus. The relative intensity can thus slightly vary depending on the direction of crystal growth, the size of particles, the measurement conditions or the like and therefore should not be strictly interpreted.

It is also common knowledge that the 2*θ* value of each peak in powder X-ray diffraction slightly fluctuates depending on the sample conditions and the measurement conditions. The present invention encompasses not only crystals in which the diffraction angles (2*θ* (°)) of peaks in powder X-ray diffraction completely coincide but also crystals in which the diffraction angles (2*θ* (°)) of all or a part of the peaks coincide within a range of ±0.3°.

In a thermogravimetric-differential thermal analysis chart, "endothermic peak" in a DTA curve is represented by the temperature at peak top (peak top) or "extrapolation initiation temperature". "Extrapolation initiation temperature" means the intersection between the onset point or the offset point in the DTA curve and extrapolation of the baseline and is also referred to as "extrapolated onset temperature".

"Extrapolation initiation temperature" is a temperature at the starting point of the peak, and it means exothermic or endothermic starting temperature calculated by the extrapolation. The peak top and the extrapolation initiation temperature in a thermogravimetric-differential thermal analysis chart may also slightly fluctuate depending on the measurement conditions. For example, in general, the temperature may fluctuate within a range of ±5°C. That is, the crystal specified by the above peaks encompasses crystals which coincide within a range of ±5°C.

In the present invention, "around" used in the thermal analysis means a range of ±5°C.

In a ¹³C solid-state NMR spectrum chart, since the chemical shift values (δ (ppm)) can vary somewhat depending on the measurement conditions, the identity of the crystal form should be recognized even when the chemical shift values are slightly different, and the crystal within such an error range is also included in the present invention. The error of the chemical shift value of, for example, ±0.5 ppm is conceivable. That is, the crystal specified by a chemical shift value (δ (ppm)) encompasses crystals which coincide within a range of ±0.5 ppm. Further, the peak intensity may change or the peak may appear or disappear due to a difference in the rotation frequency or the measurement device.

### EXAMPLES

The contents of the present invention are further illustrated in more detail by way of the following Examples and Test Examples. However, the present invention is not limited thereto.

### (Example 1)

### 1-{[(4aR,6R,8aR)-2-Amino-3-cyano-8-methyl-4,4a,5,6,7,8,8a,9-octahydrothieno[3,2-g]quinolin-6-yl]carbonyl}-3-[2-(dimethylamino)ethyl]-1-propylurea sesquisuccinate monohydrate (Form I crystal of salt (A-1))

To 1-{[(4aR,6R,8aR)-2-amino-3-cyano-8-methyl-4,4a,5,6,7,8,8a,9-octahydrothieno[3,2-g]quinolin-6-yl]carbonyl}-3-[2-(dimethylamino)ethyl]-1-propylurea (22.00 g), 102.8 g of acetone was added, and the mixture was suspended, heated and stirred at an external temperature of 52°C to dissolve the mixture. Activated carbon (2.2 g) was added to the solution, and the mixture was stirred for 10 minutes. The suspension was hot-filtered and it was washed with 35.2 g of acetone. Furthermore, 220.0 g of acetone was added to the mixture, and the reaction liquid was heated to an external temperature of 52°C and stirred. Then, 44.0 g of water was added to the reaction liquid. Separately, 8.73 g of succinic acid was dissolved in a mixed solution of 156.1 g of acetone and 19.8 g of water. The succinic acid solution was added dropwise to the reaction liquid over about 10 minutes. The dropping funnel was washed with a mixed solution of 17.4 g of acetone and 2.2 g of water, and the washing liquid was added dropwise to the reaction liquid. The reaction liquid was stirred at an internal temperature of 50°C for 1 hour and cooled to 15°C over 30 minutes. The reaction liquid was stirred at an external temperature of 10°C for 2 hours, and crystals were collected by filtration. The crystals were washed with 52.8 g of acetone two times. The obtained wet crystals were dried under reduced pressure at 50°C for 37 hours and returned to room temperature under reduced pressure over 3 hours. The crystals were stored under an atmosphere for 24 hours to give the crystals of the title compound (27.75 g).
¹H-NMR(DMSO-d6) (δ (ppm)): 0.85(3H, t, J=7.4 Hz), 1.32 (1H, ddd, J=12.2 Hz, 12.2 Hz, 12.2 Hz), 1.42-1.57 (2H, m), 1.57-1.70 (1H, m), 1.89-2.00 (2H, m), 2.20-2.13 (1H, m), 2.13-2.28 (2H, m), 2.21(3H, s), 2.24 (6H, s), 2.35-2.48 (1H, m), 2.40 (6H, s), 2.46 (2H, t, J=6.4 Hz), 2.81-2.96(2H, m), 3.00-3.12 (1H, m), 3.21-3.33 (2H, m), 3.47-3.66 (2H, m), 6.99 (2H, s), 8.50-8.90 (1H, br).

### Single-crystal X-ray structural analysis

A single crystal of the Form I of 1-{[(4aR,6R,8aR)-2-amino-3-cyano-8-methyl-4,4a,5,6,7,8,8a,9-octahydrothieno[3,2-g]quinolin-6-yl]carbonyl}-3-[2-(dimethylamino)ethyl]-1-propylurea sesquisuccinate monohydrate (salt (A-1)) was prepared and subjected to X-ray structural analysis.

### (Preparation of single crystal and preparation of measurement)

1-{[(4aR,6R,8aR)-2-Amino-3-cyano-8-methyl-4,4a,5,6,7,8,8a,9-octahydrothieno[3,2-g]quinolin-6-yl]carbonyl}-3-[2-(dimethylamino)ethyl]-1-propylurea (30 g) was suspended in a mixed solution of 84 g of acetone and 110 g of water, and 11.9 g of succinic acid was added. The mixture was heated to an internal temperature of 51°C to dissolve the mixture. The solution was added to 720 mL of acetone at 50°C under stirring while passing through a filter paper. The vessel and the filter paper were washed with 110 g of acetone. The Form I crystal of the salt (A-1) in an amount of 6 mg was added to a mixed liquid of the filtrate and the washing liquid to start crystallization. The mixture was stirred at an internal temperature of 50°C for 1 hour and stirred under ice-cooling for 2 hours 30 minutes. The obtained suspension was filtered, and the solid on the filter paper was washed with 72 g of acetone two times. The obtained wet crystals were dried under reduced pressure at 50°C for 18 hours to give the crystal of the title compound (43.2 g). The powder X-ray diffraction of the obtained crystal was measured by the method of Test Example 1, and it was confirmed that the crystal form was the same as that of the crystal obtained in Example 1. Single crystals were collected from the powder, cut and shaped with razors, mounted in microloops with grease and snap frozen in a gas stream of a low temperature device.

The X-ray diffraction data were measured and acquired under the following measurement conditions using XtaLAB P200 MM007 (Rigaku Corporation).

### (Measurement Conditions)

X-ray source: CuKα
Wavelength: 1.54187 Å
Tube voltage and tube current: 40 kV, 30 mA
Measurement temperature: -100°C
Crystal size: 0.15×0.08×0.04 mm
Vibration angle: 1°
Exposure time: 2 seconds/sheets:
   Total number of measured sheets: 1637
   Total measurement time: 55 minutes

### (Data analysis program)

Data measurement, diffraction data processing: Crystal Clear
Structural analysis and refinement method: Crystal Structure, SIR2011, SHELXL2013

### (Measurement results)

The obtained measurement results are shown in Table 1.

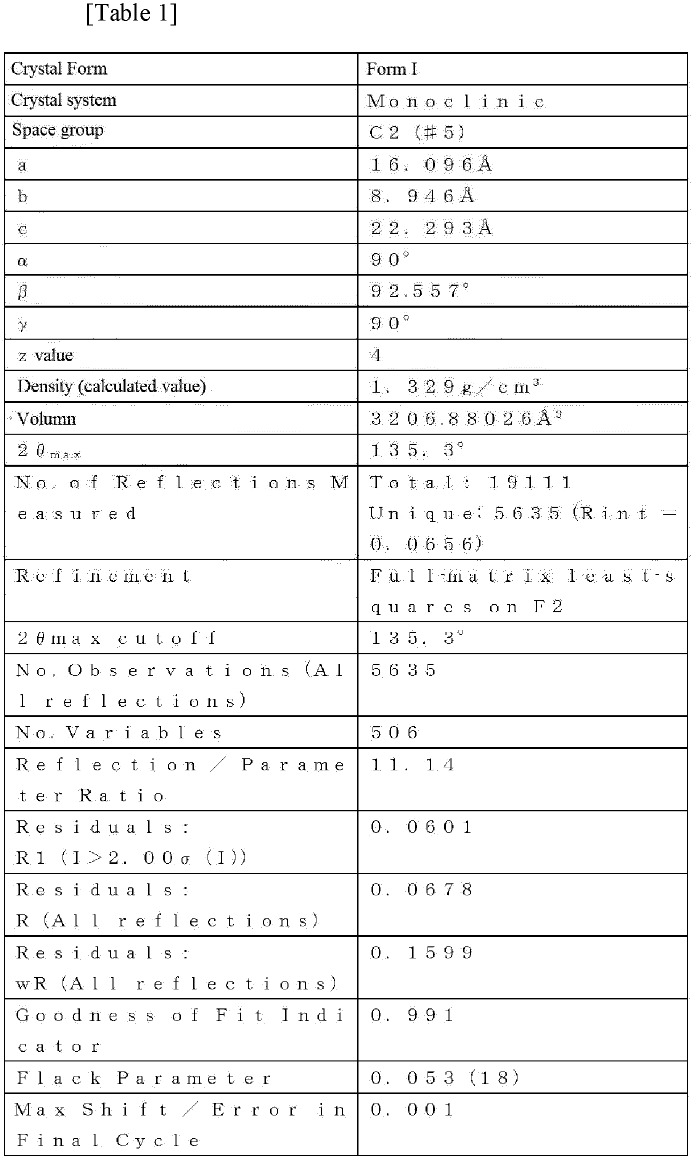

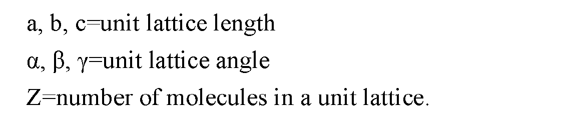

The elemental analysis was performed using a CHN automatic analyzer vario EL (Elemental).

### (Elemental Analysis Results (C, H, N))

Theoretical value: 52.40%, 7.07%, 13.10%
Measured value: 52.25%, 7.07%, 12.98%.

From the above measurement results, the Form I crystal of the salt (A-1) is a crystal which has a monoclinic crystal system, a space group of C2 and a z value of 4 and in which 2 molecules of 1-{[(4aR,6R,8aR)-2-amino-3-cyano-8-methyl-4,4a,5,6,7,8,8a,9-octahydrothieno[3,2-g]quinolin-6-yl]carbonyl}-3-[2-(dimethylamino)ethyl]-1-propylurea, 3 molecules of succinic acid and 2 molecules of water are present in the asymmetric unit.

### (Examples 2)

### 1-{[(4aR,6R,8aR)-2-Amino-3-cyano-8-methyl-4,4a,5,6,7,8,8a,9-octahydrothieno[3,2-g]quinolin-6-yl]carbonyl}-3-[2-(dimethylamino)ethyl]-1-propylurea sesquisuccinate salt (Form II crystal of salt (A-1))

1-{[(4aR,6R,8aR)-2-Amino-3-cyano-8-methyl-4,4a,5,6,7,8,8a,9-octahydrothieno[3,2-g]quinolin-6-yl]carbonyl}-3-[2-(dimethylamino)ethyl]-1-propylurea sesquisuccinate salt in an amount of 121 mg was added to a mixed solvent (3 mL) of 1,4-dioxane/water (volume ratio 1:1), and the mixture was heated to 60°C, dissolved and filtered. The obtained filtrate was lyophilized. To the obtained powder, 2.5 mL of n-heptane was added, and the mixture was heated to 60°C in a suspended state and stirred for 1 hour. After stirring at room temperature for 1 day, the solid was collected by filtration and dried under reduced pressure at 40°C to give crystals of the title compound (92 mg).
¹H-NMR(MeOH-d4) (δ (ppm)): 0.96 (3H, t, J=7.2 Hz), 1.50 (1H, ddd, J=12.0 Hz, 12.4 Hz, 12.4 Hz), 1.57-1.70 (2H, m), 1.75-1.88 (1H, m), 2.03-2.26 (3H, m), 2.31-2.46 (4H, m), 2.46-2.56 (7H, m), 2.58-2.66 (1H, m), 2.71 (6H, s), 2.98-3.10 (4H, m), 3.15-3.25 (1H, m), 3.52-3.59 (2H, m), 3.62-3.72 (2H, m)

### (Examples 3)

### 1-{[(4aR,6R,8aR)-2-Amino-3-cyano-8-methyl-4,4a,5,6,7,8,8a,9-octahydrothieno[3,2-g]quinolin-6-yl]carbonyl}-3-[2-(dimethylamino)ethyl]-1-propylurea monosuccinate salt (Form I crystal of salt (A-2))

1-{[(4aR,6R,8aR)-2-Amino-3-cyano-8-methyl-4,4a,5,6,7,8,8a,9-octahydrothieno[3,2-g]quinolin-6-yl]carbonyl}-3-[2-(dimethylamino)ethyl]-1-propylurea (357 mg) was dissolved in 3.5 mL of acetone under heating at an internal temperature of 50 to 55°C, and then the solution was cooled to an internal temperature of 4°C in an ice bath to prepare a reaction liquid. Separately, succinic acid (94 mg) was dissolved in a mixed solvent (0.3 mL) of acetone/water (volume ratio 1:1) under heating at an internal temperature of 55°C. To the cooled reaction liquid, the succinic acid solution was added dropwise, and the mixture was concentrated to dryness under reduced pressure. A mixed solvent (1.35 mL) of acetone/water (volume ratio 1:1) was added to the residue, and the mixture was dissolved under heating at an internal temperature of 55°C. The mixture was stirred at room temperature to precipitate crystals. After stirring for 10 minutes, a mixed solvent (0.45 mL) of acetone/water (volume ratio 1:1) was further added to the suspension, and the mixture was stirred at room temperature for 10 minutes. Furthermore, 7.2 mL of acetone was added in 3 portions in 2 hours, and the mixture was stirred at room temperature for 1 hour. The precipitated crystals were collected by filtration, washed with a small amount of acetone and dried under reduced pressure at room temperature for 1 hour to give crystals of the title compound (393 mg).
¹H-NMR(MeOH-d4) (δ (ppm)): 0.96 (3H, t, J=7.6 Hz), 1.50 (1H, ddd, J=12.4, 12.4, 12.4 Hz), 1.59-1.69 (2H, m), 1.73-1.86 (1H, m), 2.03-2.21 (3H, m), 2.30-2.42 (4H, m), 2.43-2.55 (5H, m), 2.57-2.70 (7H, m), 2.92 (2H, t, J=6.8 Hz), 2.97-3.07 (2H, m), 3.49-3.55 (2H, m), 3.63-3.82 (2H, m).

### (Example 4)

### Pharmaceutical composition containing Form I crystal of salt (A-1)

To a mixture (78.1 mg) of mannitol (49 parts), crystalline cellulose (50 parts) and sodium stearyl fumarate (1 part), the Form I crystal (8.7 mg) of the salt (A-1) of Example 1 was added and mixed well at room temperature to obtain a pharmaceutical composition containing 10% of the salt (A-1).

### (Test Example 1) Measurement of Powder X-ray Diffraction

The powder X-ray diffraction of the Form I crystal of the salt (A-1) and the Form I crystal of the salt (A-2) was measured under the following measurement conditions using a powder X-ray diffractometer X'Pert Pro MPD (Panalytical, Spectris Co., Ltd.) after the crystals were lightly mortar-pulverized to pulverize coarse particles.

### (Measurement Conditions)

Radiation source: CuKα rays (CuKα1 and CuKα2), 1.5418 Å
Tube voltage: 45 kV
Tube current: 40 mA
Data analysis software: X'Pert HighScore (Panalytical, Spectris Co., Ltd.)
Data analysis method (peak search): Minimum significance (1.00), Minimum peak tip (0.01, 2*θ* (°)) and the maximum peak tip (1.00, 2*θ* (°)), peak base (2.00, 2*θ* (°)), method (smoothed peak top) (2*θ* (°))

The powder X-ray diffraction of the Form II crystal of the salt (A-1) was measured under the following measurement conditions using a powder X-ray diffractometer SmartLab (Rigaku Corporation) after the crystals were lightly mortar-pulverized to pulverize coarse particles.

### (Measurement Conditions)

Radiation source, wavelength: CuKα rays (CuKα1 and CuKα2), 1.5418 Å
Tube voltage: 40 kV
Tube current: 50 mA
Data analysis software: SmartLabStudio II (Rigaku Corporation)
Data analysis method (peak definition): Peak position (peak top position, diffraction angles upon irradiation with CuKα1 and CuKα2), peak height (excluding background)

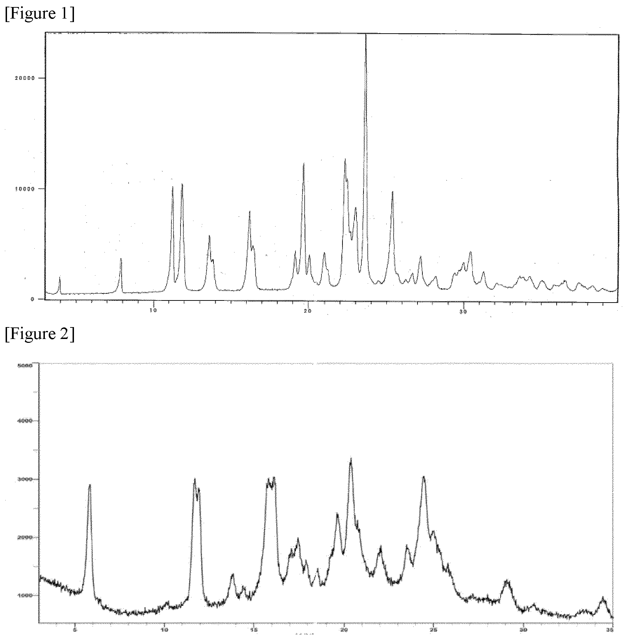

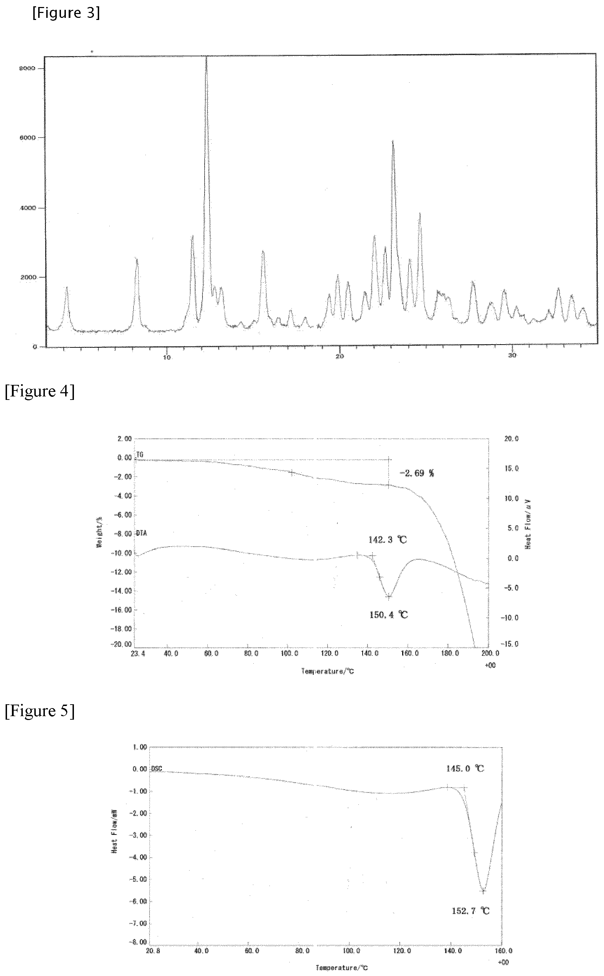

The diffraction diagram of the Form I crystal of the salt (A-1) is shown in Figure 1, and the diffraction angles (2*θ* (°)) of representative diffraction peaks and the relative intensities (%) of the diffraction peaks are shown in Table 2. In addition, the diffraction diagram of the Form II crystal of the salt (A-1) is shown in Figure 2, and the diffraction angles (2*θ* (°)) of representative diffraction peaks and the relative intensities (%) of the diffraction peaks are shown in Table 3. In addition, the diffraction diagram of the Form I crystal of the salt (A-2) is shown in Figure 3, and the diffraction angles (2*θ* (°)) of representative diffraction peaks and the relative intensities (%) of the diffraction peaks are shown in Table 4.

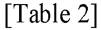

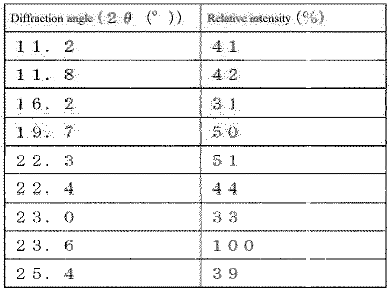

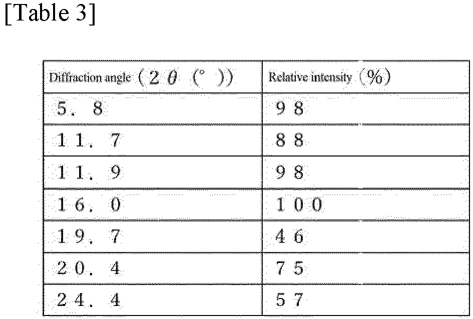

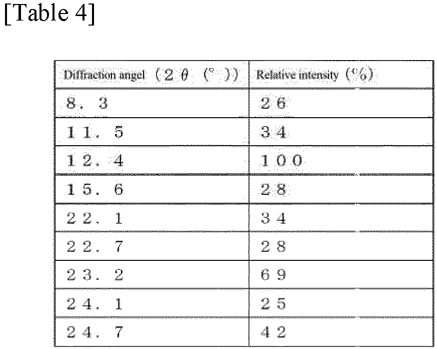

For the identification of the Form I crystal of the salt (A-1), for example, the peak sets of diffraction angles (2*θ* (°)) below can be used. One peak set is 11.2±0.3 and 11.8±0.3. Yet another peak set is 11.2±0.3, 11.8±0.3 and 16.2±0.3. Yet another peak set is 11.2±0.3, 11.8±0.3 and 23.6±0.3. Yet another peak set is 11.2±0.3, 11.8±0.3, 23.6±0.3 and 25.4±0.3. Yet another peak set is 11.2±0.3, 11.8±0.3, 16.2±0.3, 19.7±0.3, 22.3±0.3, 22.4±0.3, 23.0±0.3, 23.6±0.3 and 25.4±0.3.

For the identification of the Form II crystal of the salt (A-1), for example, the peak sets of diffraction angles (2*θ* (°)) below can be used. One peak set is 5.8±0.3, 20.4±0.3 and 24.4±0.3. Yet another peak set is 5.8±0.3, 11.7±0.3, 11.9±0.3, 16.0±0.3, 20.4±0.3 and 24.4±0.3.

For the identification of the Form I crystal of the salt (A-2), for example, the peak sets of diffraction angles (2*θ* (°)) below can be used. One peak set is 8.3±0.3, 12.4±0.3, 15.6±0.3 and 23.2±0.3. Yet another peak set is 8.3±0.3, 11.5±0.3, 12.4±0.3, 15.6±0.3, 22.1±0.3, 22.7±0.3, 23.2±0.3, 24.1±0.3 and 24.7±0.3.

### (Test Example 2) Measurement of Thermal Analysis

The thermal analysis was performed using a differential thermal balance TG-DTA TG8120 (Rigaku Corporation) under the following measurement conditions under a nitrogen gas atmosphere.

### (Measurement Conditions)

Heating rate: 10°C/min
Reference material: Aluminium oxide
Atmosphere: under a nitrogen gas stream

The TG-DTA measurement diagram of the Form I crystal of the salt (A-1) is shown in Figure 4.

The endothermic peak of the Form I crystal of the salt (A-1): a broad endothermic peak at 80 to 130°C, around 150°C (a peak top (extrapolation initiation temperature around 142°C (melting))
Mass decrease: around 23°C to 150°C: 2.7%

The DSC chart of the Form I crystal of the salt (A-1) is shown in Figure 5.

The endothermic peak of the Form I crystal of the salt (A-1): a broad endothermic peak at 80 to 130°C, around 153°C (peak top) (extrapolation initiation temperature around 145°C).

### (Test Example 3) Measurement of ¹³C Solid-State NMR Spectrum

The ¹³C solid-state NMR spectra of the Form I crystal of the salt (A-1) and the Form I crystal of the salt (A-2) were obtained by measuring the specimen which was filled in a solid-state NMR spectrum measurement rotor of an inner diameter of 3.2 mm, under the following measurement conditions to obtain an NMR chart.

### (Measurement Conditions)

NMR instrument: 600 MHz AVANCE III (Bruker)
Probe: Cross-polarization magic angle rotation (CP/MAS) accessory
Contact time: 3 milliseconds
Recycling delay: 5 seconds
1H pulse: 3 microseconds
Rotation speed: 15 kHz
Integration number: 2048
Chemical shift correction: Glycine was referred to. (δ=176.46 ppm for C=O resonance)

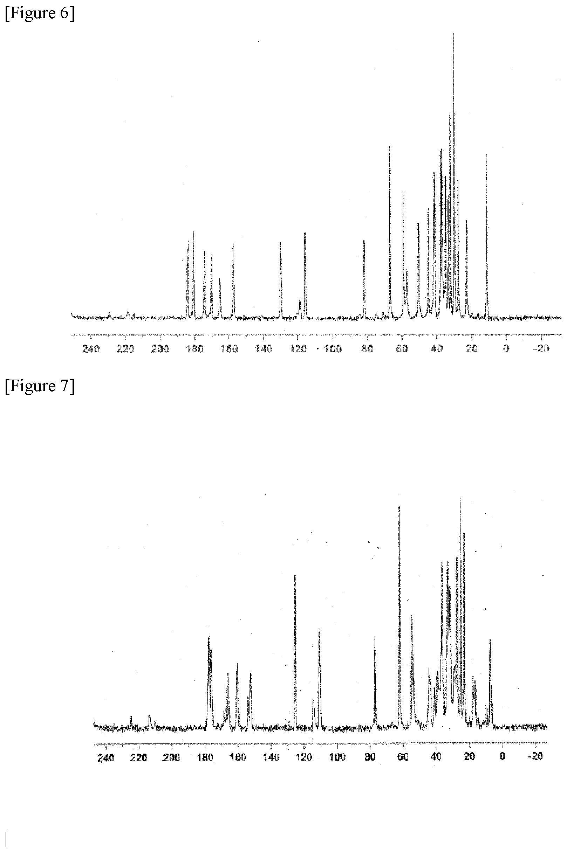

The solid-state NMR spectrum of the Form I crystal of the salt (A-1) obtained in Example 1 is shown in Figure 6, and the chemical shifts (ppm) are shown in Table 5. Also, the solid-state NMR spectrum of the Form I crystal of the salt (A-2) obtained in Example 3 is shown in Figure 7, and the chemical shifts (ppm) are shown in Table 6.

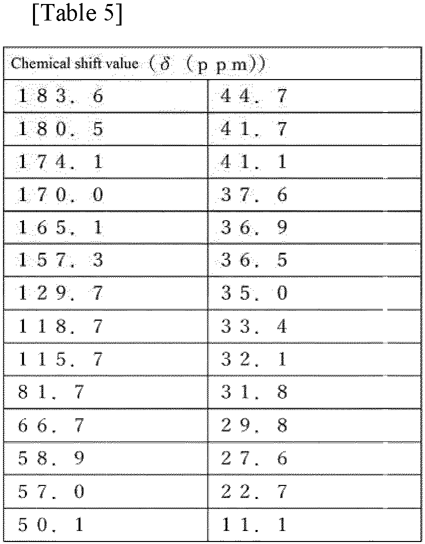

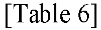

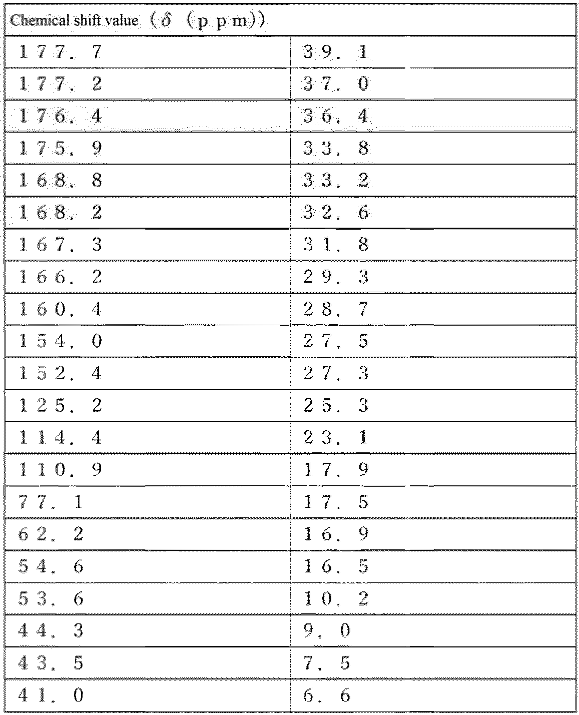

For the identification of the Form I crystal of the salt (A-1), for example, the sets of chemical shift values (δ (ppm)) of a ¹³C solid-state NMR spectrum below can be used. One set is 183.6±0.5, 180.5±0.5, 174.1±0.5, 170.0±0.5, 165.1±0.5 and 157.3±0.5. Another set is 183.6±0.5, 180.5±0.5, 174.1±0.5, 170.0±0.5, 165.1±0.5, 157.3±0.5, 129.7±0.5, 115. 7±0.5, 81.7±0.5, 66.7±0.5, 58.9±0.5, 22.7±0.5 and 11.1±0.5. Another set is 183.6±0.5, 180.5±0.5, 174.1±0.5, 170.0±0.5, 165.1±0.5, 157.3±0.5, 129.7±0.5, 115.7±0.5, 58.9±0.5, 22.7±0.5 and 11.1± 0.5. Yet another set is 183.6±0.5, 180.5±0.5, 174.1±0.5, 170.0±0.5, 165.1±0.5, 157.3±0.5, 129.7±0.5, 118.7±0.5, 115.7±0.5, 81.7±0.5, 66.7±0.5, 58.9±0.5, 57.0±0.5, 50.1±0.5, 44.7±0.5, 41.7±0.5, 41.1±0.5, 37.6±0.5, 36.9±0.5, 36.5±0.5, 35.0±0.5, 33.4±0.5, 32.1±0.5, 31.8±0.5, 29.8±0.5, 27.6±0.5, 22.7±0.5 and 11.1±0.5.

For the identification of the Form I crystal of the salt (A-2), for example, the sets of chemical shift values (δ (ppm)) below can be used. One set is 177.7, 176.4, 166.2, 160.4, 154.0 and 152.4. Another set is 177.7, 177.2, 176.4, 175.9, 166.2, 160.4, 154.0 and 152.4. Another set is 177.7, 177.2, 176.4, 175.9, 168.8, 168.2, 167.3, 166.2, 160.4, 154.0 and 152.4. Another set is 177.7, 176.4, 166.2, 160.4, 154.0, 152.4, 125.2, 114.4, 110.9, 77.1, 62.2, 54.6 and 7.5. Yet another set is 177.7, 177.2, 176.4, 175.9, 168.8, 168.2, 167.3, 166.2, 160.4, 154.0, 152.4, 125.2, 114.4, 110.9, 77.1, 62.2, 54.6, 53.6, 44.3, 43.5, 41.0, 39.1, 37.0, 36.4, 33.8, 33.2, 32.6, 31.8, 29.3, 28.7, 27.5, 27.3, 25.3, 23.1, 17.9, 17.5, 16.9, 16.5, 10.2, 9.0, 7.5 and 6.6.

In the present invention, the Form I crystal of the salt (A-1) can also be identified by combining the above peaks of powder X-ray diffraction, a ¹³C solid-state NMR spectrum and a thermogravimetric-differential thermal analysis chart.

As an embodiment for identifying the Form I crystal of the salt (A-1), the following embodiments (c1) to (c4) are illustrated, for example.
(c1) A powder X-ray diffraction diagram having peaks at diffraction angles (2*θ* (°)) of 11.2±0.3 and 11.8±0.3; and a ¹³C solid-state NMR spectrum chart having peaks at chemical shift values (δ (ppm)) of 183.6±0.5, 180.5±0.5, 174.1±0.5, 170.0±0.5, 165.1±0.5 and 157.3±0.5.
(c2) A powder X-ray diffraction diagram having peaks at diffraction angles (2*θ* (°)) of 11.2±0.3 and 11.8±0.3; and a thermogravimetric-differential thermal analysis chart having an extrapolation initiation temperature of an endothermic peak at around 150°C (hereinafter, "extrapolation initiation temperature" is referred to as "onset temperature").
(c3) A ¹³C solid-state NMR spectrum chart having peaks at chemical shift values (δ (ppm)) of 183.6±0.5, 180.5±0.5, 174.1±0.5, 170.0±0.5, 165.1±0.5 and 157.3±0.5; and a thermogravimetric-differential thermal analysis chart having an onset temperature of an endothermic peak at around 150°C.
(c4) A powder X-ray diffraction diagram having peaks at diffraction angles (2*θ* (°)) of 11.2±0.3 and 11.8±0.3; a ¹³C solid-state NMR spectrum chart having peaks at chemical shift values (δ (ppm)) of 183.6±0.5, 180.5±0.5, 174.1±0.5, 170.0±0.5, 165.1±0.5 and 157.3±0.5; and a thermogravimetric-differential thermal analysis chart having an onset temperature of an endothermic peak at around 150°C.

### (Comparative Example 1)

### Crystal of hydrochloride salt of compound (B)

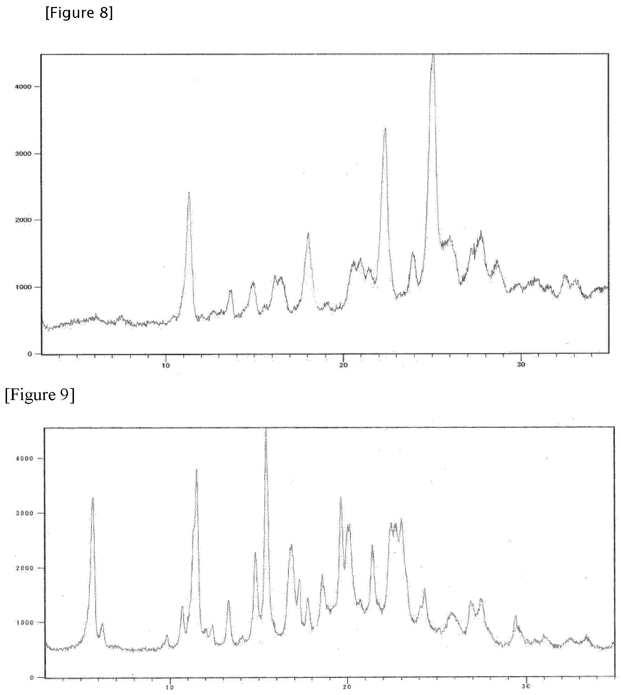

The powder X-ray diffraction of the crystal of hydrochloride salt of the compound (B) obtained by the method described in Example 4-1 of Patent literature 1 was measured in the same manner as in Test Example 1. The obtained diffraction diagram is shown in Figure 8.

### (Comparative Example 2)

### Crystal of sebacate salt of compound (B)

The compound (B) (500 mg) and sebacic acid (226 mg) were added to ethanol (3 mL), heated to 50°C and dissolved. The obtained solution was stirred at room temperature for 1 hour, and then diisopropylether (3 mL) was added thereto. The mixture was further stirred at room temperature for 3 days. The precipitated solid was collected by filtration, washed with a mixed solution of ethanol and diisopropylether (1:1), then air-dried at room temperature for 3 hours and further dried under reduced pressure at 60°C for 3 hours to give the title compound (0.4938 g). ¹H-NMR(MeOH-d4) (δ (ppm)): 0.95 (3H, t, J=7.6 Hz), 1.33 (6H, br), 1.52 (1H, q, J=12.4 Hz), 1.55-1.70 (5H, m), 1.72-1.85 (1H, m), 2.04-2.10 (1H, m), 2.10-2.21 (2H, m), 2.24 (3H, t, J=7.2 Hz), 2.29-2.40 (4H, m), 2.45 (1H, t, J=11.2 Hz), 2.51 (6H, s), 2.57-2.65 (1H, m), 2.78 (2H, t, J=6.4 Hz), 2.95-3.06 (2H, m), 3.13-3.23 (1H, m), 3.45-3.52 (2H, m), 3.63-3.81 (2H, m).

The powder X-ray diffraction of the obtained crystal of sebacate salt of the compound (B) was measured in the same manner as in Test Example 1, and the obtained diffraction diagram is shown in Figure 9.

### (Comparative Example 3)

### Crystal of adipate salt of compound (B)

The compound (B) (500 mg) and adipic acid (164 mg) were added to 3 mL of ethanol, heated to 50°C and dissolved. The obtained solution was stirred at room temperature for 1 hour, and then 3 mL of diisopropylether was added thereto. The mixture was further stirred at room temperature for 1 hour. The mixture was heated at 50°C for 10 minutes and then stirred at room temperature for 1 hour. The precipitated solid was taken out and washed with 1 mL of ethanol. The obtained solid was air-dried overnight under a laboratory atmosphere and then dried under reduced pressure at 60°C for 3 hours to give the title compound (0.3854 g).
¹H-NMR(MeOH-d4) (δ (ppm)): 0.95 (3H, t, J=7.6 Hz), 1.49 (1H, q, J=12.4 Hz), 1.58-1.69 (4H, m), 1.71-1.85 (1H, m), 2.03-2.22 (3H, m), 2.23-2.40 (6H, m), 2.40-2.54 (7H, m), 2.56-2.66 (1H, m), 2.74 (2H, t, J=6.4 Hz), 2.96-3.05 (2H, m), 3.10-3.23 (1H, m), 3.45-3.52 (2H, m), 3.62-3.81 (2H, m).

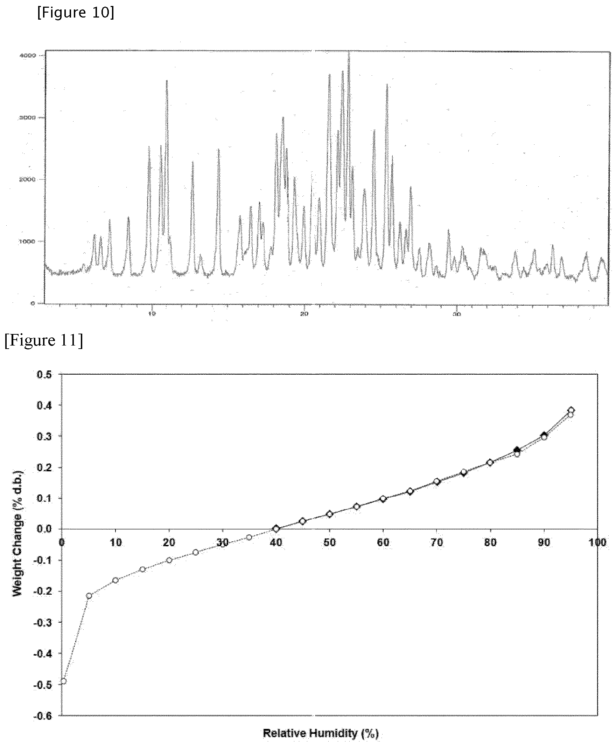

The powder X-ray diffraction of the obtained crystal of adipate salt of the compound (B) was measured in the same manner as in Test Example 1, and the obtained diffraction diagram is shown in Figure 10.

### (Test Example 4) Stability test 1

The Form I crystal of the salt (A-1), the Form I crystal of the salt (A-2), the crystal of hydrochloric acid of the compound (B), the crystal of sebacate salt of the compound (B) and the crystal of adipate salt were stored under an open condition at 60°C, and the physical stability and the chemical stability of each crystal form were examined. The powder X-ray diffractions of the samples at the start of the specimens and 2 months later were measured in the same manner as in Test Example 1, and the physical stability of the crystal forms and the amounts of the related substances were measured using the following HPLC measurement conditions, thereby observing the chemical stability. At the same time, changes in appearance were also observed. The results are shown in Table 7.

No change in crystal form of the Form I crystal of the salt (A-1) and the Form I crystal of the salt (A-2) was observed during storage under open condition at 60°C. In addition, the Form I crystal of the salt (A-1) and the Form I crystal of the salt (A-2) were chemically stable, and there was almost no change in appearance. On the other hand, the hydrochloride salt of the compound (B) and the sebacate salt of the compound (B) were changed in crystal form and were chemically unstable. In addition, the hydrochloride salt of the compound (B), the sebacate salt of the compound (B) and the adipate salt of the compound (B) were colored.

### (HPLC conditions)

Detector: Ultraviolet and visible absorptiometer/wavelength: 225 nm
Column: L-column2 ODS, 3 µm, 4.6×150 mm (manufactured by Chemicals Evaluation and Research Institute, Japan)
Column temperature: constant temperature around 30°C
Flow rate: 1.0 mL/min
Mobile phase A: a solution (pH 6.9) in which potassium dihydrogen phosphate and dipotassium hydrogen phosphate are mixed in water so as to be each 10 mmol/L
Mobile phase B: acetonitrile
Mobile phase ratios
   0 to 25 minutes: mobile phase A/mobile phase B = 79/21
   25 to 45 minutes: mobile phase A/mobile phase B = 79/21 to 25/75 (Gradient)
   45 to 50 minutes: mobile phase A/mobile phase B = 25/75
Injection volume :5 µL
Sample cooler: 4°C
Dissolution solvent: A mixed solution prepared by adding 20 parts of acetonitrile to 80 parts of a 20 mmol/L potassium dihydrogen phosphate aqueous solution adjusted to pH 3.
Sample solution: A liquid obtained by dissolving the specimen in the dissolution solvent and adjusting to about 1.0 mg/mL as the compound (B).

Excluding the peaks derived from the blank, the peak areas of the respective peaks were measured by an automatic integration method, and the values thereof were determined by an area normalization method.

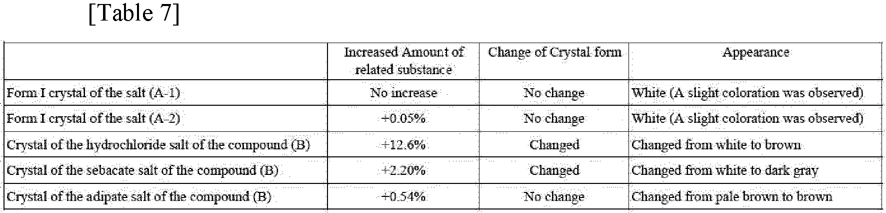

### (Test Example 5) Stability test 2

The Form I crystal of the salt (A-1), the Form I crystal of the salt (A-2), the crystal of hydrochloride salt of the compound (B), the crystal of sebacate salt of the compound (B) and the crystal of adipate salt were stored under an open condition at 40°C and 75% relative humidity, and the physical stability and the chemical stability of each crystal form were examined. The powder X-ray diffractions of the samples at the start of the specimens and 2 months later were measured in the same manner as in Test Example 1, and the physical stability of the crystal forms and the amounts of the related substances were measured using the following HPLC measurement conditions, thereby observing the chemical stability. At the same time, changes in appearance were also observed. The results are shown in Table 8.

No change in crystal form of the Form I crystal of the salt (A-1) and the Form I crystal of the salt (A-2) was observed during storage under open condition at 40°C and 75% relative humidity. In addition, the Form I crystal of the salt (A-1) and the Form I crystal of the salt (A-2) were chemically stable, and there was no change in appearance. On the other hand, the crystal forms of the hydrochloride salt of the compound (B) and the sebacate salt of the compound (B) were changed. In addition, the sebacate salt of the compound (B) was chemically unstable. The adipate salt of the compound (B) was chemically unstable, and the appearance was also colored.

### (HPLC conditions)

Detector: Ultraviolet and visible absorptiometer/wavelength: 225 nm
Column: L-column2 ODS, 3 µm, 4.6×150 mm (manufactured by Chemicals Evaluation and Research Institute, Japan)
Column temperature: constant temperature around 30°C
Flow rate: 1.0 mL/min
Mobile phase A: a solution (pH 6.9) in which potassium dihydrogen phosphate and dipotassium hydrogen phosphate are mixed in water so as to be each 10 mmol/L
Mobile phase B: acetonitrile
Mobile phase ratios
   0 to 25 minutes: mobile phase A/mobile phase B = 79/21
   25 to 45 minutes: mobile phase A/mobile phase B = 79/21 to 25/75 (Gradient)
   45 to 50 minutes: mobile phase A/mobile phase B = 25/75
Injection volume :5 µL
Sample cooler: 4°C
Dissolution solvent: A mixed solution prepared by adding 20 parts of acetonitrile to 80 parts of a 10 mmol/L potassium dihydrogen phosphate aqueous solution adjusted to pH 3.
Sample solution: A liquid obtained by dissolving the specimen in the dissolution solvent and adjusting to about 1.0 mg/mL as the compound (B).

Excluding the peaks derived from the blank, the peak areas of the respective peaks were measured by an automatic integration method, and the values thereof were determined by an area normalization method.

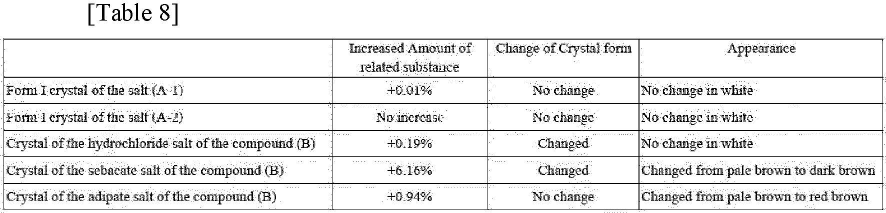

### (Test Example 6) Water adsorption and desorption test

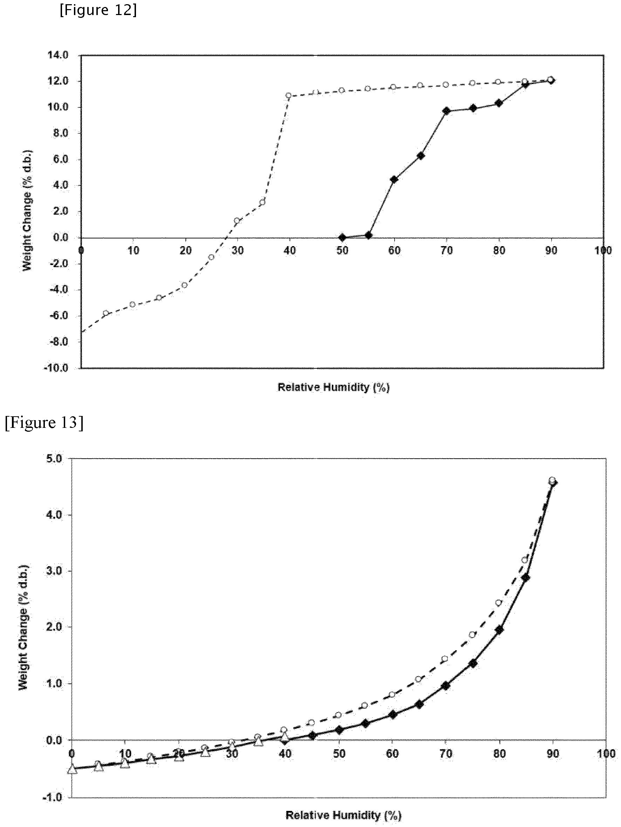

The water adsorption and desorption behaviors of the Form I crystal of the salt (A-1), the crystal of hydrochloride salt of the compound (B) and the crystal of sebacate salt of the compound (B) were measured using IGA-Sorp (manufactured by HIDEN isochema) under the following conditions. The water adsorption and desorption isotherm of the Form I crystal of the salt (A-1) is shown in Figure 11, and the water adsorption and desorption isotherm of hydrochloride salt of the compound (B) is shown in Figure 12. The water adsorption and desorption isotherm of sebacate salt of the compound (B) is shown in Figure 13.

### Specimens and amounts used for the measurement

Form I crystal of salt (A-1): 14.7 mg
Crystal of hydrochloride salt of compound (B): 10.4 mg
Crystal of sebacate salt of compound (B): 13.2 mg

### Pretreatment: Equilibration

Each specimen was placed in a water adsorption and desorption measurement device. The temperature and the humidity were set to 25°C/40% RH or 50% RH, and equilibration was conducted for 60 minutes or more to stabilize the mass.

### Measurement

The mass of each specimen subjected to mass equilibration was continuously measured while changing the relative humidity at every 5% RH in adsorption and desorption. The measurement conditions of the water adsorption and desorption measurement device were set as shown in Table 9, and the mass change amounts in the common humidity range are shown in Table 10.

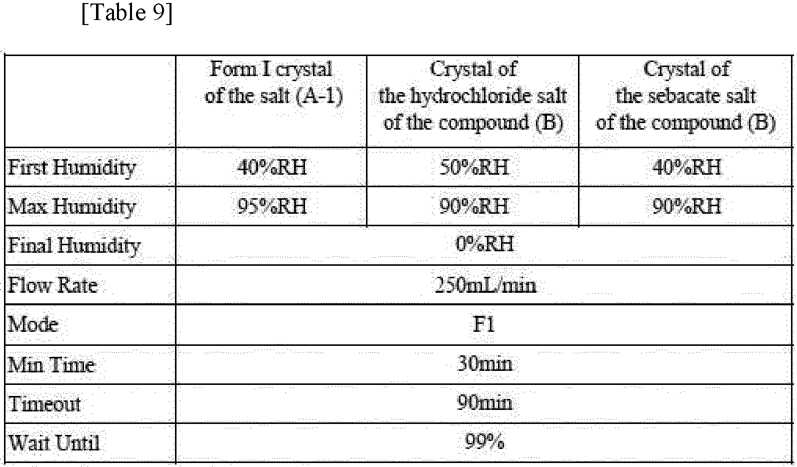

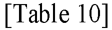

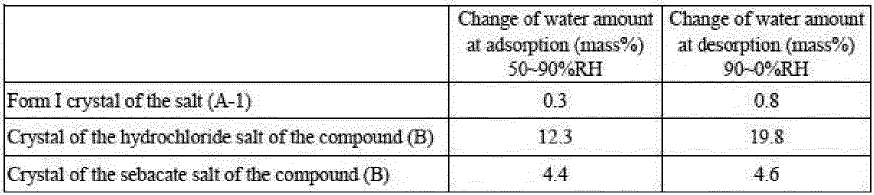

The mass % was expressed as a mass percentage of a mass change before and after adsorption (or desorption) based on a dry sample. Under the above conditions, it was found that the Form I crystal of the salt (A-1) does not exhibit hygroscopicity, whereas the hydrochloride salt of the compound (B) has hygroscopicity which is 25 to 41 times higher, and the sebacate salt of the compound (B) has hygroscopicity which is 6 to 15 times higher than that of the Form I crystal of the salt (A-1).

As described above, the succinate salt of the present invention is more preferable as a drug substance because it does not have hygroscopicity.

### (Test Example 7) Powder X-ray diffraction diagram of pharmaceutical composition

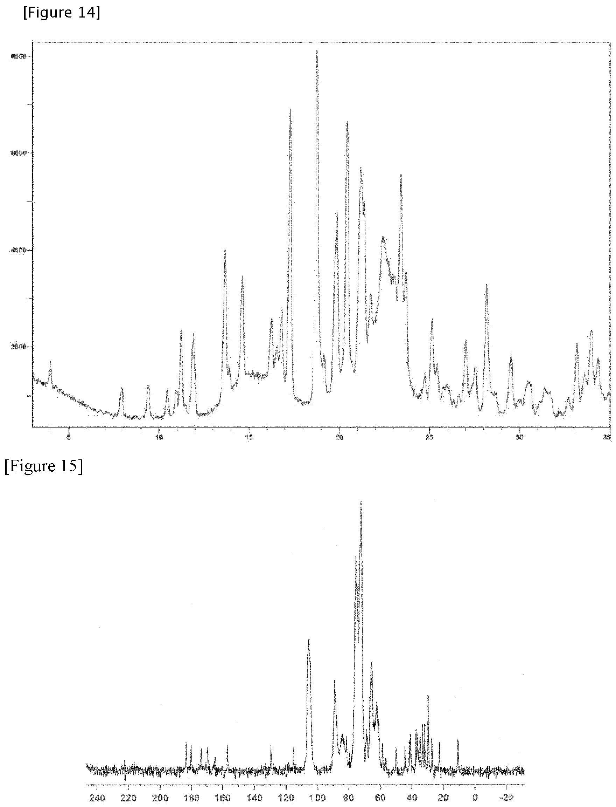

A specimen of the pharmaceutical composition was filled on a measurement plate for X-ray diffraction measurement, and the measurement was performed under the following measurement conditions to obtain a diffraction diagram. The powder X-ray diffraction diagram of the pharmaceutical composition obtained in Example 4 is shown in Figure 14, and the diffraction angles (2*θ* (°)) of representative diffraction peaks are shown in Table 11.

### (Measurement Conditions)

Powder X-ray diffractometer: SmartLab (Rigaku Corporation)
Radiation source: CuKα ray
Tube voltage: 40 kV
Tube current: 50 mA
Data analysis software: SmartLabStudio II (Rigaku Corporation)
Data analysis method (peak definition): Peak position (peak top position, diffraction angles upon irradiation with CuKα1 and CuKα2), peak height (excluding background)

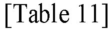

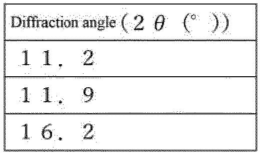

For the identification of the Form I crystal of the salt (A-1) in the pharmaceutical composition, for example, the peak sets of diffraction angles (2*θ* (°)) below can be used. One peak set is 11.2±0.3 and 11.9±0.3. Yet another peak set is 11.2±0.3, 11.9±0.3 and 16.2±0.3.

### (Test Example 8) ¹³C Solid-state NMR spectrum of pharmaceutical composition

A specimen of pharmaceutical composition was filled on a solid-state NMR spectrum measurement rotor of an inner diameter of 3.2 mm, and the measurement was performed under the following measurement conditions to obtain a solid-state NMR spectrum chart. The solid-state NMR spectrum of the pharmaceutical composition obtained in Example 4 is shown in Figure 15, and the chemical shifts (ppm) derived from the Form I crystal of the salt (A-1) are shown in Table 12.

### (Measurement Conditions)

Nuclear Magnetic Resonance Apparatus: 600 MHz AVANCE III (Bruker)
Probe: Cross-polarization magic angle rotation (CP/MAS) accessory
Contact time: 3 milliseconds
Recycling delay: 5 seconds
1H pulse: 3 microseconds
Rotation speed: 15 kHz
Integration number: 2048
Chemical shift correction: Glycine was referred to. (δ=176.46 ppm for C=O resonance)

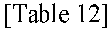

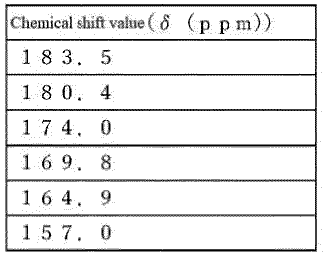

For the identification of the Form I crystal of the salt (A-1) in the pharmaceutical composition, for example, the peak sets of ¹³C solid-state NMR spectral chemical shifts (ppm) below can be used. One peak set is 183.5±0.5 and 180.4±0.5. Yet another peak set is 183.5±0.5, 180.4±0.5, 174.0±0.5, 169.8±0.5, 164.9±0.5 and 157.0±0.5.

### INDUSTRIAL APPLICABILITY

The succinate salt of the present invention has excellent storage stability and other physical properties, is useful as a drug substance and is suitable for industrial production of a pharmaceutical product.

## Claims

1. A succinate salt of 1-{[(4aR,6R,8aR)-2-amino-3-cyano-8-methyl-4,4a,5,6,7,8,8a,9-octahydrothieno[3,2-g]quinolin-6-yl]carbonyl}-3-[2-(dimethylamino)ethyl]-1-propylurea.

2. The succinate salt according to claim 1, wherein the succinate salt is represented by the following formula (A-1) or formula (A-2).

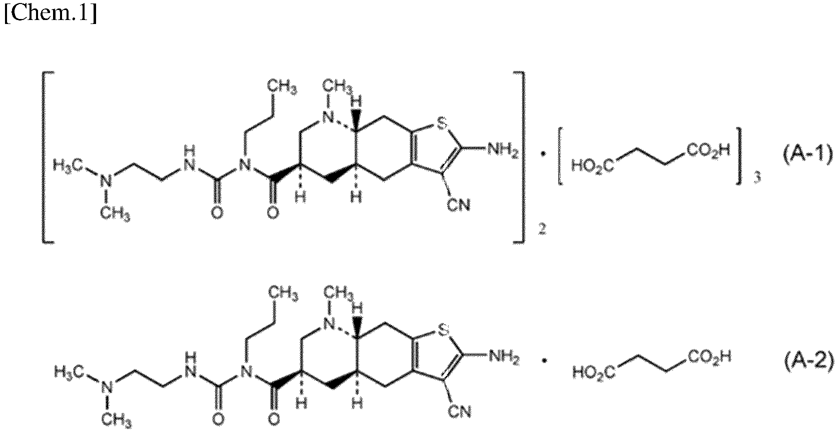

3. The succinate salt according to claim 1, wherein the succinate salt is represented by the following formula (A-1).

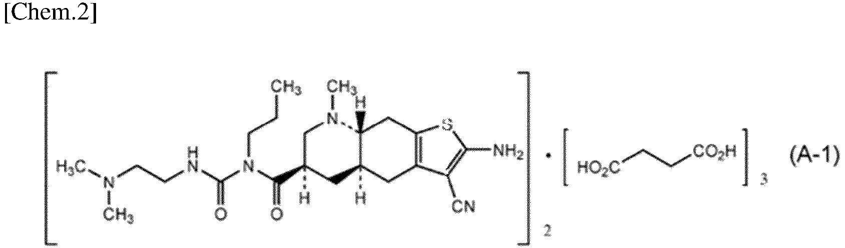

4. The succinate salt according to claim 1, wherein the succinate salt is represented by the following formula (A-2).

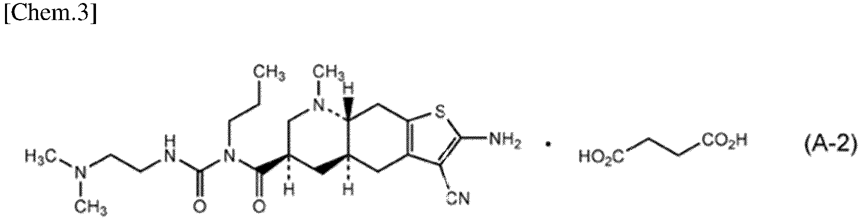

5. The succinate salt according to claim 3, which is crystalline.

6. The succinate salt according to claim 4, which is crystalline.

7. The succinate salt according to claim 5, which has peaks at diffraction angles (2*θ* (°)) of 11.2±0.3 and 11.8±0.3 in a powder X-ray diffraction diagram.

8. The succinate salt according to claim 5, which has an endothermic peak at around 150°C in a thermogravimetric-differential thermal analysis chart.

9. The succinate salt according to claim 5, which has peaks at chemical shift values (δ (ppm)) of 183.6±0.5, 180.5±0.5, 174.1±0.5, 170.0±0.5, 165.1±0.5 and 157.3±0.5 in a ¹³C solid-state NMR spectrum chart.

10. The succinate salt according to claim 5, which is **characterized by** 2 or 3 physical features selected from the group consisting of the following (a1) to (a3):
(a1) a powder X-ray diffraction diagram having peaks at diffraction angles (2*θ* (°)) of 11.2±0.3 and 11.8±0.3;
(a2) a ¹³C solid-state NMR spectrum chart having peaks at chemical shift values (δ (ppm)) of 183.6±0.5, 180.5±0.5, 174.1±0.5, 170.0±0.5, 165.1±0.5 and 157.3±0.5; and
(a3) a thermogravimetric-differential thermal analysis chart having an onset temperature of an endothermic peak at around 150°C.

11. The succinate salt according to claim 6, which has peaks at diffraction angles (2*θ* (°)) of 8.3±0.3, 12.4±0.3, 15.6±0.3 and 23.2±0.3 in a powder X-ray diffraction diagram.

12. The succinate salt according to claim 6, which has peaks at chemical shift values (δ (ppm)) of 177.7, 176.4, 166.2, 160.4, 154.0 and 152.4 in a ¹³C solid-state NMR spectrum chart.

13. A pharmaceutical composition comprising the succinate salt according to any of claims 1 to 12.

14. The pharmaceutical composition according to claim 13, for use in a method for the treatment or prevention of Parkinson's disease, restless legs syndrome or hyperprolactinemia.

15. A pharmaceutical composition comprising the succinate salt according to claim 1 having peaks at diffraction angles (2*θ* (°)) of 11.2±0.3 and 11.9±0.3 in a powder X-ray diffraction diagram of the pharmaceutical composition and at least one additional excipient.

16. A pharmaceutical composition comprising the succinate salt according to claim 1 having peaks at chemical shift values (δ (ppm)) of 183.5±0.5 and 180.4±0.5 in a ¹³C solid-state NMR spectrum chart of the pharmaceutical composition and at least one additional excipient.

## Patentansprüche

1. Succinatsalz von 1-{[(4aR,6R,8aR)-2-Amino-3-cyano-8-methyl-4,4a,5,6,7,8,8a,9-octahydrothieno[3,2-g]chinolin-6-yl]carbonyl}-3-[2-(dimethylamino)ethyl]-1-propylharnstoff.

2. Succinatsalz nach Anspruch 1, wobei das Succinatsalz durch die folgende Formel (A-1) oder Formel (A-2) dargestellt ist.

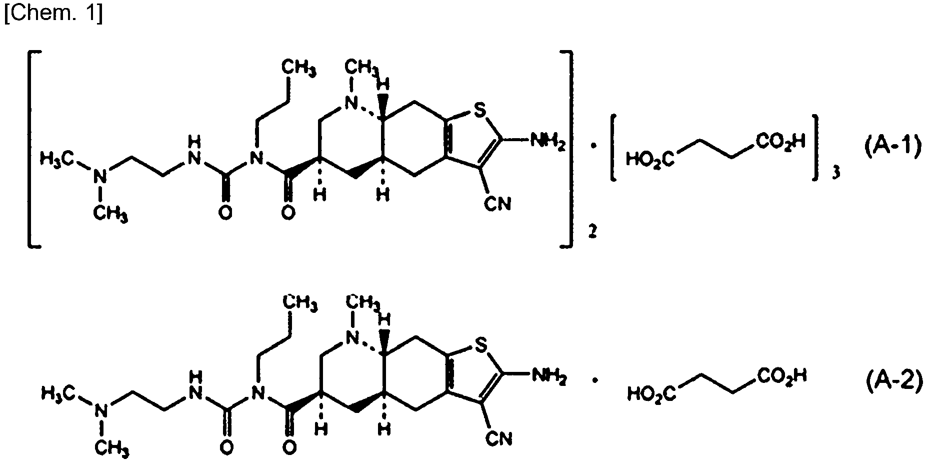

3. Succinatsalz nach Anspruch 1, wobei das Succinatsalz durch die folgende Formel (A-1) dargestellt ist.

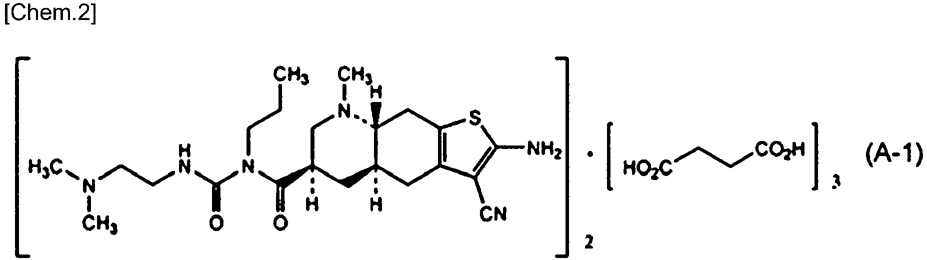

4. Succinatsalz nach Anspruch 1, wobei das Succinatsalz durch die folgende Formel (A-2) dargestellt ist.

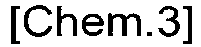

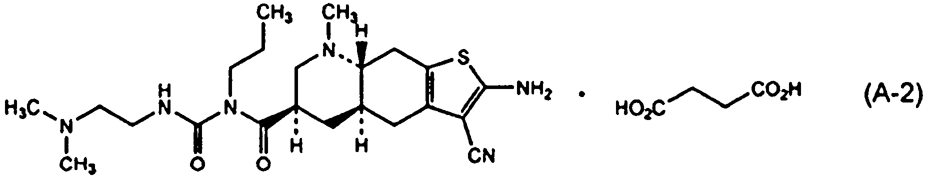

5. Succinatsalz nach Anspruch 3, welches kristallin ist.

6. Succinatsalz nach Anspruch 4, welches kristallin ist.

7. Succinatsalz nach Anspruch 5, welches Peaks bei Beugungswinkeln (2*θ* (°)) von 11,2±0,3 und 11,8±0,3 in einem Pulver-Röntgenbeugungsdiagramm aufweist.

8. Succinatsalz nach Anspruch 5, welches einen endothermen Peak bei etwa 150 °C in einem thermogravimetrischen Differentialthermoanalyse-Diagramm aufweist.

9. Succinatsalz nach Anspruch 5, welches Peaks bei chemischen Verschiebungswerten (δ (ppm)) von 183,6±0,5, 180,5±0,5, 174,1±0,5, 170,0±0,5, 165,1±0,5 und 157,3±0,5 in einem ¹³C-Festkörper-NMR-Spektrumdiagramm aufweist.

10. Succinatsalz nach Anspruch 5, welches durch 2 oder 3 physikalische Merkmale gekennzeichnet ist, ausgewählt aus der Gruppe bestehend aus den folgenden (a1) bis (a3):
(a1) ein Pulver-Röntgenbeugungsdiagramm mit Peaks bei Beugungswinkeln (2*θ* (°)) von 11,2±0,3 und 11,8±0,3;
(a2) ein ¹³C-Festkörper-NMR-Spektrumdiagramm mit Peaks bei chemischen Verschiebungswerten (δ (ppm)) von 183,6±0,5, 180,5±0,5, 174,1±0,5, 170,0±0,5, 165,1±0,5 und 157,3±0,5; und
(a3) ein thermogravimetrisches Differentialthermoanalyse-Diagramm mit einer Anfangstemperatur eines endothermen Peaks bei etwa 150 °C.

11. Succinatsalz nach Anspruch 6, welches Peaks bei Beugungswinkeln (2*θ* (°)) von 8,3±0,3, 12,4±0,3, 15,6±0,3 und 23,2±0,3 in einem Pulver-Röntgenbeugungsdiagramm aufweist.

12. Succinatsalz nach Anspruch 6, welches Peaks bei chemischen Verschiebungswerten (δ (ppm)) von 177,7, 176,4, 166,2, 160,4, 154,0 und 152,4 in einem ¹³C-Festkörper-NMR-Spektrumdiagramm aufweist.

13. Pharmazeutische Zusammensetzung, umfassend das Succinatsalz nach einem der Ansprüche 1 bis 12.

14. Pharmazeutische Zusammensetzung nach Anspruch 13 zur Verwendung in einem Verfahren zur Behandlung oder Vorbeugung von Parkinson-Krankheit, Restless-Legs-Syndrom oder Hyperprolaktinämie.

15. Pharmazeutische Zusammensetzung, umfassend das Succinatsalz nach Anspruch 1 mit Peaks bei Beugungswinkeln (2*θ* (°)) von 11,2±0,3 und 11,9±0,3 in einem Pulver-Röntgenbeugungsdiagramm der pharmazeutischen Zusammensetzung und mindestens einem zusätzlichen Hilfsstoff.

16. Pharmazeutische Zusammensetzung, umfassend das Succinatsalz nach Anspruch 1 mit Peaks bei chemischen Verschiebungswerten (δ (ppm)) von 183,5±0,5 und 180,4±0,5 in einem ¹³C-Festkörper-NMR-Spektrumdiagramm der pharmazeutischen Zusammensetzung und mindestens einem zusätzlichen Hilfsstoff.

## Revendications

1. Sel de succinate de 1-{[(4aR,6R,8aR)-2-amino-3-cyano-8-méthyl-4,4a,5,6,7,8,8a,9-octahydrothiéno[3,2-g]quinoléin-6-yl]carbonyl}-3-[2-(diméthylamino)éthyl]-1-propylurée.

2. Sel de succinate selon la revendication 1, dans lequel le sel de succinate est représenté par la formule (A-1) ou la formule (A-2) suivante.

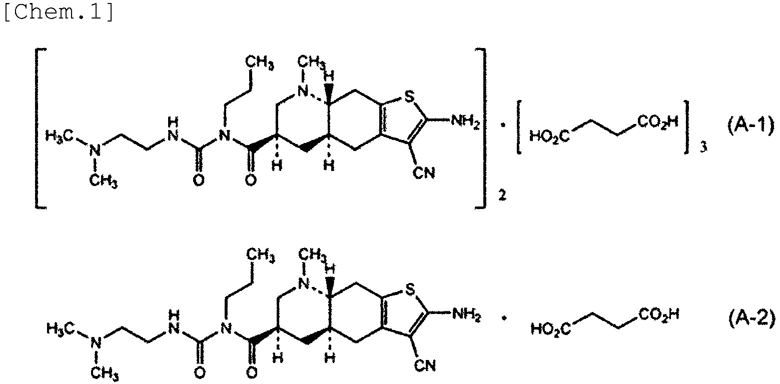

3. Sel de succinate selon la revendication 1, dans lequel le sel de succinate est représenté par la formule (A-1) suivante.

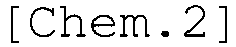

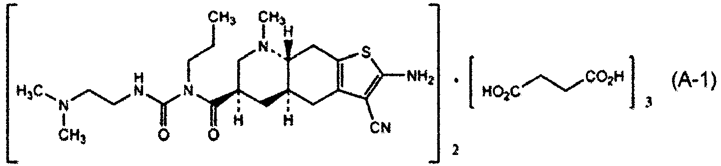

4. Sel de succinate selon la revendication 1, dans lequel le sel de succinate est représenté par la formule (A-2) suivante.

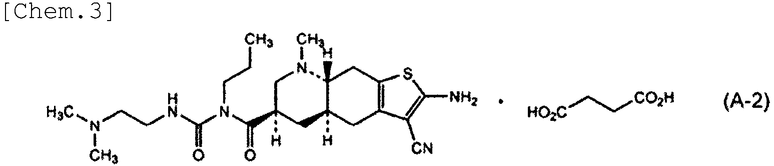

5. Sel de succinate selon la revendication 3, qui est cristallin.

6. Sel de succinate selon la revendication 4, qui est cristallin.

7. Sel de succinate selon la revendication 5, qui présente des pics aux angles de diffraction (2*θ* (°)) de 11,2±0,3 et 11,8±0,3 sur un diagramme de diffraction des rayons X sur poudre.

8. Sel de succinate selon la revendication 5, qui présente un pic endothermique à environ 150 °C sur un graphique d'analyse thermique thermogravimétrique différentielle.

9. Sel de succinate selon la revendication 5, qui présente des pics aux valeurs de déplacement chimique (δ(ppm)) de 183,6±0,5, 180,5±0,5, 174,1±0,5, 170,0±0,5, 165,1±0,5 et 157, 3±0, 5 sur un graphique de spectre RMN ¹³C à l'état solide.

10. Sel de succinate selon la revendication 5, qui est **caractérisé par** 2 ou 3 caractéristiques physiques sélectionnées dans le groupe consistant en (a1) à (a3) suivants :
(a1) un diagramme de diffraction des rayons X sur poudre présentant des pics aux angles de diffraction (2*θ* (°)) de 11,2±0,3 et 11,8±0,3 ;
(a2) un graphique de spectre RMN ¹³C à l'état solide présentant des pics aux valeurs de déplacement chimique (δ(ppm)) de 183,6±0,5, 180,5±0,5, 174,1±0,5, 170,0±0,5, 165,1±0,5 et 157,3±0,5 ; et
(a3) un graphique d'analyse thermique thermogravimétrique différentielle présentant une température de début d'un pic endothermique à environ 150°C.

11. Sel de succinate selon la revendication 6, qui présente des pics aux angles de diffraction (2*θ* (°)) de 8,3±0,3, 12,4±0,3, 15,6±0,3 et 23,2±0,3 sur un diagramme de diffraction des rayons X sur poudre.

12. Sel de succinate selon la revendication 6, qui présente des pics aux valeurs de déplacement chimique (δ(ppm)) de 177,7, 176,4, 166,2, 160,4, 154,0 et 152,4 sur un graphique de spectre RMN ¹³C à l'état solide.

13. Composition pharmaceutique comprenant le sel de succinate selon l'une quelconque des revendications 1 à 12.

14. Composition pharmaceutique selon la revendication 13, pour une utilisation dans un procédé de traitement ou de prévention de la maladie de Parkinson, du syndrome des jambes sans repos ou de l'hyperprolactinémie.

15. Composition pharmaceutique comprenant le sel de succinate selon la revendication 1 présentant des pics aux angles de diffraction (2*θ* (°)) de 11,2±0,3 et 11,9±0,3 sur un diagramme de diffraction des rayons X sur poudre de la composition pharmaceutique et au moins un excipient supplémentaire.

16. Composition pharmaceutique comprenant le sel de succinate selon la revendication 1 présentant des pics aux valeurs de déplacement chimique (δ(ppm)) de 183,5±0,5 et 180,4±0,5 sur un graphique de spectre RMN ¹³C à l'état solide de la composition pharmaceutique et au moins un excipient supplémentaire.
